(19) Europäisches Patentamt
European Patent Office
Office européen des brevets

(11) Veröffentlichungsnummer: **0 412 938 A2**

(12) **EUROPÄISCHE PATENTANMELDUNG**

(21) Anmeldenummer: 90810586.9

(22) Anmeldetag: 02.08.90

(51) Int. Cl.⁵: **A61L 2/18**, G02C 13/00

(30) Priorität: 05.08.89 DE 3926003
04.11.89 DE 3936765

(43) Veröffentlichungstag der Anmeldung:
13.02.91 Patentblatt 91/07

(84) Benannte Vertragsstaaten:
AT BE CH DE DK ES FR GB GR IT LI NL SE

(71) Anmelder: **Koller, Bruno**
**Grünwaldweg 8**
**D-6470 Büdigen 1(DE)**

(72) Erfinder: **Koller, Bruno**
**Grünwaldweg 8**
**D-6470 Büdigen 1(DE)**

(74) Vertreter: **Kerr, Andrew**
**Postfach Finkelerweg 44**
**CH-4144 Arlesheim(CH)**

(54) **Pflegesystem für Kontaktlinsen.**

(57) Das technische Problem und die Zielsetzung zum Tragen von Hydroweich-Kontaktlinsen liegt darin, wie man diese täglich ohne Pflegemittelgifte auf das Auge bekommt, damit die Augen gesund und die Hydrolinsen auf Dauer verträglich bleiben. Zu diesem Zweck müssen die schwammigen Hydrolinsen an der Oberfläche und in ihrem Innern täglich absolut sauber bleiben und zuverlässig gegen Keime behandelt werden, um dem Infektrisiko entgegenzuwirken.

Da von der Hydrolinse aufgeschwommene Pflegemittelgifte sehr hartnäckig festsitzen (elektrolytisch bedingt) und diese tage-und nächtelang langsam an das Auge abgeben, ist es notwendig, ein Pflegeverfahren aufzubauen, das frei ist von Pflegemittelgiften, das mit physikalischen Kräften arbeitet und nur tränenfilmidentische Stoffe enthält. Eine sanfte Gründlichkeit gibt der Hydrolinse eine besonders lange Lebensdauer und ermöglicht das gewünschte giftfreie Aufsetzen nach zuverlässiger Keimbehandlung.

Die Linsen werden mit einem hypertonisierten Tensid-Linsenreiniger, der von der Weichlinse nicht aufgesaugt wird, sondern dieser bereits Schmutz und Wasser entzieht, gereinigt und in einem Hydroweichlinsenaufbewahrer, der frei von augenfremden Stoffen ist, der stärker hypertonisiert ist als der Reiniger, so stark, dass er auch gleichzeitig konserviert, aufbewahrt. Vor dem Aufsetzen werden die Linsen mit isotonischer Salzlösung, die frei von augenfremden Stoffen ist gespült.

EP 0 412 938 A2

## PFLEGESYSTEM FÜR KONTAKTLINSEN

Die Erfindung betrifft ein Verfahren zur Pflege weicher Kontaktlinsen und dafür geeignete Mittel.

Man unterscheidet zwei Pflegeverfahren für hydrophile Weichlinsen:

1. Das eine Verfahren kann aufgrund seiner Kompliziertheit und der Gefährlichkeit seiner Chemikalien nur im Labor angewendet werden (siehe DE-A-24 43 147). Solche Verfahren kommen immer dann zur Anwendung, wenn der Linsenträger Schmutzrückstände an seinen hydroweichen Linsen hat, mit denen er nicht mehr fertig wird, und die eine Weiterverwendung der Linsen unmöglich machen. Solche Linsen bringt der Linsenträger seinem Lieferanten bzw. Linsenanpasser zur Aufarbeitung. In einzelnen Fällen können solche Linsen auch mit resistenten Kulturen überlastet sein. Deshalb sollte für diesen Intensiv-Pflegedienst der Linsenträger auch sein Etui zur Spezialbehandlung dem Linsenanpasser überlassen. Sehr oft sind Verfärbungen an den Linsen in gleicher Art auch am Etui festzustellen. Die Ursache hierfür kann z.B. darin liegen, dass nach dem Linsenabsetzen Kosmetika an den Fingerkuppen haften, die Fingerkuppen nun nicht mit Seife abgewaschen werden, sondern die Linsen hantiert und abgereinigt werden, so dass Kosmetika von den schwammartig hydroweichen Linsen aufgesaugt werden und so auch an das Etui und in das Etui verschleppt werden. Dieses Beispiel zeigt, dass ein richtiges Pflegesystem auch an richtige Gebrauchshinweise gebunden sein muss, um sich in der Praxis bewähren zu können.

2. Das andere Verfahren besteht aus Fertig-Pflegemitteln zum Verkauf an Linsenträger, welche täglich angewendet werden sollen und alle praktischen Anforderungen bestens erfüllen sollen.

Bekannte Pflegeverfahren, ihre Vorzüge und Nachteile:

A) Das Pflegen der hydroweichen Linsen nach dem alten Kochverfahren (Hitzedesinfektion)

Als Anfang der sechziger Jahre von Professor Wichterle, Prag, die ersten hydroweichen Kontaktlinsen auf den Markt kamen, begann auch die Entwicklung von Pflegemethoden. Der Mangel an Fachkenntnissen in der Gestaltung, speziell der individuellen Gestaltung der Linsen, und das Fehlen richtiger, perfekter Linsenpflegemittel-Systeme bremste den Vertrieb dieser wertvollen Linsen erheblich. Diese beiden Problemstellungen sind in stark geminderter Form heute noch auf dem Kontaktlinsenmarkt vorhanden. Um dem toxischen Problem aus dem Weg zu gehen, werden von den Linsenanpassern in der BRD noch zu 50% hartflexible Linsen angepasst. Meist werden die hydroweichen Linsen von den Linsenhändlern (Anpassern) dem Kunden nicht zur Probe angeboten. Im Gegenteil, sie werden dem Klienten ausgeredet, denn bei einer vergleichenden Anprobe werden die hydroweichen Linsen fast immer vom Linsenträger bevorzugt. Die Furcht vor den toxischen Problemen hemmt die Linsenanpasser, denn die hydroweiche Linse wirkt wie ein Schwamm, der die verwendeten, reinigenden, konservierenden, desinfizierenden Pflegechemikalien speichert und während des Tragens langsam an das Auge abgibt, und zwar täglich. Chemikalienbelastung, also Krankheit für das Auge, ist von vornherein das Risiko. Ohne Zweifel hatten alle Systempflegemethoden versucht, dieses Problem zu reduzieren oder sogar zu lösen. Allein die Tatsache, dass es immer noch toxische Ausfälle gibt und das Problem von vornherein gefürchtet wird, zeigt, dass auch in diesem Punkt noch Wichtiges zu tun ist.

Das alte Kochverfahren war wie folgt:

1) Mit physiologischer Kochsalzlösung wurden die hydroweichen Linsen nach dem Tragen abgespült, in ein kochfestes Etui gegeben, mit physiologischer Kochsalzlösung wurde das Etui aufgefüllt, verschlossen und das Etui 10 Minuten in kochendes Wasser gelegt. Die Linsen wurden nach dem Abkühlen wieder aufgesetzt.

Vorteile: Giftige Chemikalien kamen nicht zur Anwendung.

Nachteile: Vor dem Kochen wurde das Etui innen und aussen nicht gereinigt; das gleiche unterblieb auch bei der Hydrolinse. Schmutz kochte sich am Etui, im Etui, an der Linsenoberfläche und in der Hydroweichlinse fest. Nach dem Abkühlen verengen sich die Poren und hielten das denaturierte Eiweiss und das Fett der Tränenlösung fest. Die Linsen wurden schnell immer unverträglicher und waren meist mit dem Etui innerhalb von 6 Monaten unbrauchbar und auch durch Intensivreinigung nicht mehr zu retten. Sie wurden steifer und verfärbt. Dieses einfache, billige Verfahren war letztlich sehr kostspielig. Durch das denaturierte Eiweiss gab es vermutlich auch toxische Reaktionen. Die Linsen neigten dazu, sich nach dem Aufsetzen am Auge festzusaugen, Rötung und Brennen zu verursachen, weil durch Kochen und Verdunstung die Salzlösung im Etui hypertonische wurde.

2) Dann wurden manuelle Hartlinsenreiniger eingesetzt, um die Linsen vor dem Kochen zu reinigen, um ein Festkochen der Tränenfilmrückstände an und in der Linse zu vermeiden. Bei diesem Verfahren kochte sich nun der mit Konservierungsstoffen und gallertartigen Verdickungsmitteln belastete Linsenreiniger in die Weichlinse. Graubelagbildung, Porenverstopfungen und toxische Reaktionen waren die Folgen.

3) Alsdann kamen leicht konservierte Kochsalzlösungen auf den Markt, die zum Abkochen verwendet wurden. Die Keimbehandlung war so verbessert, die toxischen Probleme blieben aber bestehen. All diese Varianten litten zudem an dem unpraktischen Nachteil, dass ein tägliches Abkochen nötig war. Es machte das Verfahren aufwendig.

Im Moment sind hauptsächlich Peroxid-Pflegesysteme auf dem Markt. Die hydroweichen Linsen werden zur Desinfektion un Reinigung in 3% Peroxid eingelegt, 10-20 Minuten, also nicht zu lange, weil das Peroxid ausser dem Schmutz auch die Linsen angreift. Der vermeintliche Vorteil liegt darin, dass der Linsenträger glauben kann, das manuelle Linsenreinigen entfalle, die Methode sei einfacher.

Die Nachteile sind aber:

Eine konservierende Lagerung fehlt. Schon nach rund einer Woche zeigen solche Linsen bei vielen Linsenträgern einen Graufilm, der sich täglich verstärkt. Schmutz an den Linsen ist aber der Nährboden für Krankheitserreger, Schmutzfilme können das Auge röten und Unverträglichkeit verursachen. Weil dieses Verfahren dem Linsenträger Bequemlichkeit, also selbsttätige Reinigung verspricht, so wird bei diesem System einmal wöchentlich das Baden der Linse in einer Lösung empfohlen, in der eine Enzymreinigungstablette aufgelöst wird. Diese ist äusserst angriffig an Schmutz und Linse und übertrifft die 3%ige Peroxidlösung. Gelangen Rückstände dieser Tablette auf das Auge, so sind bleibende Schäden, z.B. Narben auf der Hornhaut möglich. Dieser neue Sehfehler kann nur mit Hartkontaktlinsen korrigiert werden. Die Rückstände dieser Tablette haften derart intensiv, dass auch durch Abreiben der Linsen, selbst mit verschiedenen Linsenreinigern, auch nach einer halben Stunde der Glitschfilm, der ja auch zudem an der Linse einen Film bildet (Schmutz), nicht losgelöst werden kann. Nur das Einweichen über Nacht schafft Verbesserung. Diese Tablette schafft zudem sehr leicht toxische Probleme für das Auge. Das Gleiche gilt auch für das Peroxid, denn nur ein sehr langes Einweichen in einer Salzlösung, die frei ist von augenfremden Stoffen, entgiftet die Hydrolinse auch von innen.

Bei allen Pflegesystemen konnte bisher festgestellt werden, dass sich am Auge heftige toxische Reaktionen bildeten, wenn chemisch desinfiziert und anschliessend in ungiftiger, pH- und osmotisch neutraler Salzlösung eingeweicht wurde, und zwar auch noch nach Einweichzeiten, die über Nacht wirkten. Haben Linsenträger so behandelte Linsen anschliessend Tag und Nacht getragen, so wurden die Augen erst weiss und die Linsen erst dann völlig verträglich, wenn diese ca. 3 Tage und 3 Nächte auf dem Auge waren. Nach praktischen Erfahrungen vcn Kontaktlinsenanpassern gilt dies für alle chemisch desinfizierenden Hydroweichlinsen-Pflegesysteme. Dieser praktische Nachteil ergab sich auch in einigen Fällen bei dem Pflegeverfahren gemäss DE-A-33 18 211.

Bei dem genannten Peroxidsystem ergeben sich die toxischen Ausfälle aus verschiedenen Gründen:

Der verwendete Biokatalysator (pflanzliches Enzym) ist toxisch. Nicht alle Enzyme müssen durch das Peroxid zerstört werden. Die zerstörten Enzyme sind ein chemisches Umwandlungsprodukt, genannt Katalase. Katalase ist toxisch und in Arzneimitteln verschreibungspflichtig, in Kosmetika verboten. Katalase ist auch als Ablagerung an der Linse als Schmutz zu bewerten. Die vorgeschlagene Einweichzeit von 10 Minuten genügt nicht, um Peroxid aus der hydroweichen Linse auszuschwemmen. Auch das Katalysieren benötigt mindestens 10 Minuten. Ein weiteres Beispiel: Eine hartflexible Linse, die in 3%igem Peroxid gelegen hat, ist auch, wenn man sie mit einem Reiniger ca. 30 Minuten abreinigt und gründlich abspült, noch unverträglich und macht das Auge rot und brennt heftig. Sie muss mindestens 1-2 Stunden eingeweicht sein, um wieder verträglich zu sein. Nach einer solchen Einweichzeit muss man aber noch nicht eine ungiftige Linse haben, denn Verträglichkeit beinhaltet den Begriff individueller Tcleranz.

Die zu lösenden Aufgaben sind im Detail vielfältig und sollen hier aufgeführt werden.

Zu wünschen wäre ein Pflegesystem, dass die hydroweichen Linsen nach dem Tragen tadellos reinigt, sehr zuverlässig konserviert, sterilisiert und vollkommen giftfreies Linsenaufsetzen garantiert. Für diese Aufgaben, d.h. Reinigung, Aufbewahrung und Spülung wären völlig giftfreie Lösungen notwendig, zumindest sollten auch der Reiniger und der Aufbewahrer frei von augenfremden Stoffen sein. Bisher kannte man dieses Prinzip nur bei den Linsenabspülern.

Bei der Frage, ob auch ein Hydroweichlinsen-Reiniger frei von augenfremden Stoffen sein kann, so muss dies verneint werden, z.B. eine Ultraschallreinigung entfettet nicht. Das Auge sondert Öl und Talg ab. Auch das Fett vom Schweiss der Finger und des Gesichtes, von Gesichts- und Handcreme oder fettigen Kosmetika kann immer nach dem Absetzen an der Linse sein. Eine verfettete Linse kann weder chemisch noch physikalisch desinfiziert werden, noch kann eine solche Linse Reizfreiheit oder ein gutes Sehen ermöglichen. Wo bereits Fett ist, sammelt sich auch wieder Fett an. Die Fettreinigung ist mit Peroxid nicht richtig zu bewerkstelligen. Das ist der Hauptgrund, warum bei diesem Verfahren die Tablettenreinigung nachgeschoben werden musste. Um das ungiftige Linsentragen und die Sauberkeit zu verbessern, müsste dann ein zweiter Abspüler (nicht toxisch, frei von Katalase) eingesetzt werden. Das Verfahren wäre dann an Kompliziertheit nicht mehr zu übertreffen.

Fettlösende Wirkstoffe sind z.B. Buthylacetat, Reinigungsbenzin, Alkohole, etc. Sie sind explosiv, lösen Fingernagellack, etc. Sie sind zum Mitnehmen in Hand- und Aktentaschen unbrauchbar. Lösungen, angesetzt mit Verdünnungsmitteln, wie Polyvinylakohol, Hydroxycellulose, etc. beseitigen nur leichte Verfettungen durch Einpacken in Verbindung mit Abreiben. Diese Lösungen verstopfen gern die Poren der Hydrolinsen und bilden leicht Graubelag.

Der Erfindung liegt demnach die Aufgabe zugrunde, den Trägern von hydrophilen Kontaktlinsen ein Pflegesystem in die Hand zu geben, dass in der täglichen Anwendungspraxis in allen Aufgabenbereichen Ergebnisse bringt, die so hochwertig wie möglich sind: Sauberkeit von innen und an den Oberflächen, konservierence Lagerung, Desinfektion und ein giftfreies Aufsetzen. Dieses Verfahren soll der hydrophilen Linse eine möglichst lange Verwendbarkeitszeit ermöglichen und möglichst schnelle, richtige, unkomplizierte Anwendung sicherstellen.

Erfindungsgemäss wird dies erreicht durch ein Verfahren zur Pflege von weichen Kontaktlinsen, bei dem die Linse in einem hypertonisierten Tensid-Reiniger ohne Zusatz von Verdickungsmitteln und Phosphaten gereinigt, in einer wässrigen, von augenflüssigkeitsfremden Stoffen freien und mit 20 Gew.% im Tränenfilm enthaltener Salze hypertonisierten Lösung aufbewahrt und mit einer von augenflüssigkeitsfremden Stoffen freien, isotonischen Salzlösung nachgespült wird.

Die Tenside bieten eine absolut zuverlässige Entfettung und ein genügend leichtes Abspülen. In 5 Sekunden ist die Schmutz- und Fettlösung bereits abgeschlossen. In 5 Sekunden sind die Tenside an den Oberflächen der Linsen auch wieder abgereinigt und abgespült. Für das erfindungsgemässe Verfahren eignet sich ein Reiniger, bestehend aus einem oder mehreren Tensiden, z.B. 1% Polysorbat 20, 1% Polysorbat 80. Beide ergänzen sich in ihren Eigenschaften. Er ist frei von Phosphat (Phosphat kann Steinbildung, Niederschlag verursachen). Hierbei sind auch die Salze und Umweltgifte zu sehen, die in der Tränenlösung sein können. Auf Pufferung wird verzichtet, um die Reinigungslösung elektrolytisch unkompliziert zu halten und um den Hydroweichlinsenreiniger gleichzeitig auf sauer einzustellen. Dadurch ist er wirksamer gegen Pilze, und beim Reinigen lässt er die Hydrolinse etwas schrumpfen. Dieser Reiniger ist ganz leicht hypertonisiert und unterstützt dadurch den Schrumpfungsprozess der Hydrolinse. Die toxischen Bestandteile, wie Konservierungsstoffe und Tenside dringen so nicht zu stark in die Hydrolinse ein. Zur Hypertonisierung kommen die einwertigen Kationen Natrium und Kalium in Frage, sowie die entsprechenden Anionen Chlorid und Citrat. Alle Kombinationen sind möglich, z.B. ein Chelatbildner ist bei diesem Reiniger nicht notwendig. Zur Vorsorge kann er mit hinzugenommen werden. Dieser Reiniger ist ohne Verdickungsmittel. So kann er Linsenporen nicht verstopfen, keinen Graubelag bilden, ist leichter zu entfernen und schafft an Fingerkuppen und Etuiwänden keinen unnötigen klebrigen Belag. Eine bevorzugte Konservierung besteht aus Thimerosal und Chlorhexidin oder anderen Konservierungsstoffen, die bei Augentropfen möglich sind. Die Zugabe von EDTA ist sehr günstig.

Dieser erfindungsgemässe phosphatfreie Reiniger bekämpft nicht nur die elektrolytischen Niederschläge an hydroweichen Linsen, sondern ist auch als Reiniger von hartflexiblen Linsen erstklassig geeignet.

Anstelle dieses Reinigers, der für das Auge giftige Konservierungsstoffe enthält, hat sich folgender Reiniger als besonders vorteilhaft herausgestellt:

1. Auf herkömmliche chemische Konservierung, wie z.B. mit Thimerosal, Chlorhexidin oder vergleichbarem wird verzichtet. Eine Übertragung solcher Gifte auf das Auge, direkt oder indirekt, ist somit ausgeschlossen. Zur Konservierung werden tränenfilmidentische oder körpereigene biologische Salze verwendet, wie z.B. Natrium, Kalium, Magnesium und deren Antagonisten wie z.B. Chlorid, Acetat, Citrat, Tartrat, etc. oder einer Mischung solcher Salze bzw. Salzkombinationen. Die Salzkonzentration wird sehr stark angesetzt. Die Lösung wird also enorm stark hypertonisiert. Die Salzkonzentration kann bis um die 20% betragen, in bevorzugter Weise 10-18%.

Die angebrochene Flasche ist bei dieser starken biologischen Konservierung ohne weiteres 4 Wochen verwendbar. Abgefüllt wird bevorzugt der Bedarf von 4 Wochen, um einer längeren Verwendung vorzubeugen.

2. Damit dieser Reiniger entfettet, werden 1 oder mehrere Tenside zugesetzt, bevorzugt 1% Tween 20 und 1% Tween 89 oder z.B. Lecithin aus Gehirn und Rückenmark oder Eilecithin. Andere Zusätze fehlen vollständig , wie z.B. Verdickungsmittel. Auch auf Chelatbildner kann verzichtet werden, sowie auf Puffersalze. Dieser Reiniger ist mit tränenfilmidentischen oder körpereigenen Salzen enorm hypertonisiert, damit ist er gleichzeitig biologisch konserviert, bringt beim Abreiben bereits die Hydrolinse zum

4

Schrumpfen durch Wasserentzug. Das bedeutet, der Reiniger dringt nicht in die Hydrolinse ein, sondern reinigt bereits die Linse von innen durch Wasserentzug. Die entfettenden, toxischen, augenfremden Tenside können nicht in die Hydrolinse eindringen.

Der Reiniger besteht beispielsweise aus destilliertem Wasser und einer Zugabe eines oder mehrerer Tenside. Zur Erzeugung einer starken biologischen Konservierung hat er eine Salzzugabe von körpereigenen Salzen als individuelles Salzgemisch, bevorzugt frei von Calcium, Phosphat und Carbonat, evt. auch frei von Magnesium, bevorzugt aber nur Natrium-Chlorid. Die Hypertonisierung beträgt nun 10-18%, ideal sind 14%. Natrium kann am schlechtesten in die Hydrolinse eintreten und ist für diesen Gebrauchszweck zu bevorzugen. PH-Einstellungen sind nicht notwendig. Leichte Varianten von diesem Reiniger bestehen aus einer Zugabe von Zitronensäure oder 1-4% Kalium-Chlorat oder einem Gemisch von diesen. Zitronensäure unterstützt die Reinigung und die Konservierung; Kalium-Chlorat wirkt leicht antiseptisch.

Die beschriebenen Reiniger können auch gleichzeitig als desinfizierende Hartlinsenaufbewahrer verwendet werden.

Auch dieser Reiniger ist ideal für die rückstandlose Reinigung der hartflexiblen Linsen und aller Kontaktlinsenetuis. Nur entfettete Etuis oder Linsen können desinfiziert werden. Das gilt für das physikalische und chemische Desinfektionsprinzip.

Wenn dieser Reiniger angewendet wurde, wird die hartflexible Linse trocken gelagert in einem undichten, ventiliertem Etui, um Schimmelbildung vorzubeugen.

Rückstände an der hartflexiblen Linse werden mit weichem Papiertuch und mit einer sterilen Abspüllösung, die frei von körperfremden, augenfremden Stoffen ist, beseitigt. Sie kann alsdann in der Abspüllösung, die frei von körperfremden, augenfremden Stoffen ist, gelagert werden und alsdann mit UV-Licht sterilisiert werden. Neigt der Linsenträger zu Benetzungsproblemen, so wird der Tensidfilm nicht abgerieben, die Linse wird nur auf isotonisch gewässert in der Abspüllösung, die frei von körperfremden, augenfremden Stoffen ist. Die hartflexible Linse ist erstklassig verträglich, es sei denn, es liegt eine entsprechende Tensidallergie vor. Tenside sind Benetzungsstoffe, die die Grenzflächenspannung an hydrophoben, hartflexiblen Linsen aufhaben und so benetzend als wasseranziehend wirken und das klare Sehen ermöglichen. Z.B. Tween 20 und Tween 80 haben erstklassige Reinigungseigenschaften, verfärben nicht die Hydrolinsen und sind physiologisch enorm verträglich. Eilecithin verfärbt, reinigt schlecht, verfault leicht, ist aber enorm verträglich.

Es hat sich als besonders vorteilhaft erwisen, den vorstehend beschriebenen Reiniger gleichzeitig auch als Handwaschmittel vor dem Kontektlinsenhantieren zu verwenden.Bei dieser neuen Hygiene werden nicht der Handrücken, sondern nur die Handflächen gereinigt. Dies bringt Vorteile, weil alle Seifen entfetten und gleichzeitig eine Rückfettung bewirken, um der Haut das Gefühl der Pflege zu geben, um Trockenheit zu vermeiden. In diesem Rückfettungsfilm befinden sich allerdings die augenfremden, giftigen Stoffe der Seife. Es ist ein wesentlicher Mangel in der Kontaktlinsenhygiene, wenn dieser Rückfettungsfilm von der Hand, den Fingern auf die Linse gestempelt wird und so unbewusst das Auge in seinem Aussehen und seiner Gesundheit provoziert. Zudem kann man davon ausgehen, dass alle Seifen unsteril sind und ggf. nicht frei sind von Partikeln, die die Kontaktlinsen verkratzen können. So erscheint es nützlich, vor dem Hantieren der Kontaktlinsen ein Handwaschmittel zu verwenden, das frei von augenfremden Stoffen, oder zumindest optimal frei von augenfremden Stoffen, steril verpackt, steril gefiltert, um gleichzeitig absolute Sauberkeit zu sichern und Linsenkratzer zu verhindern, konserviert mit tränenfilmidentischen Salzen ist und ohne Rückfettung entfettet.

Das Kochen der hydroweichen Linsen als physikalische Hitzedesinfektion erzeugt keine toxischen chemischen Umwandlungsprodukte, ist aber täglich nach dem Linsentragen anzuwenden. Diese Umständlichkeit kann man reduzieren auf einen Rhythmus von einigen Tagen oder einer Woche, wenn man die richtig gereinigten Linsen nach dem zuvor beschriebenen Verfahren konservierend lagert und zwar in einer sterilen, enorm hoch konzentrierten Salzlösung, die frei von augenfremden Stoffen ist. Eine solche Lösung als Aufbewahrungslösung zu verwenden ermöglicht den Verzicht auf tägliches Abkochen der Linsen, durch zuvor richtige Reinigung, weil sie viele Keime bereits vernichtet, viele abspült und viele Keime in der Entwicklung abbremst. Dieses Prinzip der konservierenden Lagerung kennt man bereits bei Heringen und Pökelfleisch. Am zweckmässigsten wird die gereinigte Hydrolinse mit dieser Lösung nach dem Reinigen (s.o.) nachgerieben und abgespült, um den toxischen Reiniger nicht in das Linsenetui zu verschleppen. Die Hydrolinse wird nun in das Waschmaschinenetui gelagert und mit der Abspüllösung durch Drehen vorgespült. Die Abspüllösung war morgens vor dem Aufsetzen ins Etui gegeben worden zum Trimmen der Linsen auf Augenfreundlichkeit. Jezt dient sie zur Vorspülung der Linsen. Nach einigen Sekunden wird das Etui entleert und die erfindungsgemässe, hochkonzentrierte Salzlösung, die frei von augenfremden Stoffen ist, ins Etui gegeben. Der Hydrolinse wird nun fast völlig das Wasser entzogen, sowie Schmutzrückstände der Tränenlösung und des phosphatfreien Tensid-Reinigers. Die hochkonzentrierte Salzlösung (um 20 %)

EP 0 412 938 A2

ist steril und konserviert sehr stark und dient zur Kurzzeitlagerung über Nacht. Morgens wird das Etui geleert, halb gefüllt mit der isotonischen, isohydrischen Salzlösung, die frei von augenfremden Stoffen ist. Das Etui wird durchgeschüttelt, gedreht und entleert. Das Etui ist weitgehendst entsalzt. Es wird nun mit der beschriebenen Abspüllösung aufgefüllt und 10 Minuten später kann die tadellos saubere, hygienisch richtig konservierte Hydrolinse, die frei von augenfremden Stoffen ist, aufs Auge gesetzt werden. So ist der richtig passenden Hydrolinse Dauerverträglichkeit möglich.

Während der Einweichzeit von bis zu 10 Minuten kann das Etui in warmes Wasser getaucht werden. Die auf Körpertemperatur erwärmte Linse ist noch verträglicher. Die verwendete Abspüllösung verbleibt im Etui, weil abends die gereinigten Linsen darin vorgespült werden. Sollten trotzdem, was sogar angenommen werden kann, Reste der Aufbewahrungslösung mit der Linse auf die Augen gelangen, so ist dies untoxisch, ohne Bedeutung, denn der Aufbewahrer enthält nur tränenfilmidentische Elektrolyte. PH und osmotischer Druck sind physiologisch und tränenfilmidentisch und werden innerhalb von 10 Minuten des Einweichens erreicht. Eine Verwechslung der Lösungen ist ausgeschlossen für Linsenträger. Die in der Aufbewahrungslösung behandelte Linse ist zusammengeschrumpft und steif und somit nicht verwendbar. Auch eine Kostprobe der Lösungen klärt die Situation.

Die enorme Verträglichkeit und die einzigartige Langlebigkeit der Linse, einfache, schnelle, sichere Anwendung machen dieses Verfahren freundlich für Augen und Kunden. Die Pflegezeit beträgt: Abends 90 Sekunden, morgens 15 Sekunden.

Einmal wöchentlich wird das Waschmaschinenetui (mit Drehmechanismus) von innen und aussen mit Flüssigseife und weicher kleiner Zahnbürste ausgebürstet, gut gespült, die wie beschrieben gereinigte Linse ins Etui gegeben und 2-4 Minuten in der hochkonzentrierten Salzlösung belassen, alsdann wird entleert und mit der genannten Abspüllösung aufgefüllt und das verschlossene Etui mit den Linsen 10 Minuten in kochendes Wasser gelegt zur Desinfektion. Wenn man das Etui nicht öffnet, können die so behandelten Linsen 4 Wochen so gelagert werden (wie eingemachtes Obst). Für diesen Zweck kann man die Linsen auch direkt in der hochkonzentrierten Salzaufbewahrungslösung kochen und Langzeitlagerung machen. Zusätzliche Arbeitszeit mit Etuireinigung: 2 Minuten. Wichtig ist, nach dem Kochen abkühlen zu lassen, die Lösung auszugiessen und aufzufüllen mit der beschriebenen Aufbewahrungslösung und mit dem Aufsetzen 10 Minuten zu warten, um den osmotischen Druck zu regulieren. Auch dieses Vorgehen war bei dem alten Kochverfahren unbekannt, wie auch das Verwenden eines Reinigers, dessen Rückstände sich nicht festkochen können, da die Verdickungsmittel fehlen, der gleichzeitig phosphatfrei ist, und die osmotisch richtige Reinigung von aussen und innen der Hydrolinsen vor dem Kochen, das richtige Einstellen der Säure-Basen-Flut und des osmotischen Druckes von Reiniger, Aufbewahrer, Abspüllösung, die Wahl der Elektrolyte, insbesondere des Aufbewahrers, aber auch des Reinigers und Abspülers. Die neue Anweisung "vor dem Kochen das Etui entfettend mit Seife innen und aussen waschen" ist ebenfalls funktionell sehr wichtig. Schmutz darf sich nicht an der Linse festkochen. Löst sich Schmutz vom Etui, so kann dieser an der Hydrolinse und in die Hydrolinse eingekocht werden.

Statt der physikalischen Hitzedesinfektion (Abkochen) kann auch eine Kaltdesinfektion mit ultraviolettem Licht durchgeführt werden. Dieses Desinfektionsprinzip hat den Vorzug, dass keine Wasserverdunstung stattfindet, der osmotische Druck stabil bleibt, ein Nachspülen wie bei der Hitzedesinfektion entfällt. Hierzu ist ein kleines Gerät, welches an der Steckdose angeschlossen wird, ideal. Dieses UV-Sterilisationsverfahren ist ideal einzugliedern in das beschriebene Verfahren, Patentanmeldung DE 3318211A1 und DE 3315974A1 und der heutigen Patentzusatzanmeldung mit der Patentanmeldung P 39 26 003.8. Auch der Kombinationsgedanke dieses UV-Sterilisationsverfahrens mit dem beschriebenen hydrodynamischen, elektrolytischen Pflegesystems ist neu. Hierbei sei erwähnt, dass dieses Verfahren biologische Konservierung der Hydrolinsen mit tränenfilmidentischen Stoffen und in gewissen Zeitabständen Abkochen oder mit UV-Licht-Desinfizierung (auch Hartlinsen) ein besonders zuverlässiges Verfahren ist, was auch so gesehen fortschrittlich ist.

Nachdem die Spezifikation des neuen Hydrolinsenreinigers beschrieben worden ist, welcher in dieses neue physikalische Hitzedesinfektionsverfahren eingegliedert wurde, soll die Spezifikation der neuen, konservierenden Aufbewahrungslösung, die frei von augenfremden Stoffen ist, beschrieben werden:

1. Verwendet werden nur Salze, die Aufgaben im Stoffwechsel des Menschen haben, die also im natürlichen gesunden Tränenfilm vorhanden sind. Zwar ist es im Moment sehr schwierig, eine Tränenfilmanalyse von einem gesunden Menschen zu bekommen, der sich gesund ernährt. Trotzdem ist es möglich, die physiologischen, biologischen Bedingungen für den Tränenfilm bestens zuerfüllen und gleichzeitig die besten Effekte in Konservierung und Reinigung an den Linsen erhalten.

2. Folgende Elektrolyte des Tränenfilms sind ungeeignet:

A) Eisen: es verfärbt die Hydrolinsen (amberfarbig) und greift deren Oberflächen und Materialverbindungen an.

B) Schwefel: er desinfiziert erstklassig, greift aber die Linsen an, bildet sehr stark Niederschlag, ist zu reaktionsfreudig und verfärbt die Linsen (gelb).

C) Calcium: desinfiziert gut, aber bildet wie Phosphor sehr leicht Niederschlag. Da dieser Aufbewahrer auch erwärmt wird, ggf. auf Kochtemperatur, so dürfen Auskristallisierungen nicht riskiert werden. Das Inberührungkommen mit Salzen der Tränenlösung und Umweltgiften muss dabei auch berücksichtigt werden.

D) Hydrogencarbonat: dissoziiert sehr schlecht und ist daher nicht erwünscht. Es reinigt aber sehr gut. Beim Kochen wandelt es sich um in Carbonat. Carbonat reinigt sehr gut, dissoziiert ebenfalls schlecht. Beide Elektrolyte können in diese neue Aufbewahrungslösung einbezogen werden. Man kann aber auf sie verzichten.

E) Chelatbildner sind in dieser Aufbewahrungslösung, die konservierende und reinigende Aufgaben hat und zur Vorbereitung eines ungiftigen Linsentragens dient, verwendbar, jedoch nicht notwendig. Auch deswegen, weil der Reiniger und der Abspüler elektrolytisch angepasst sind.

F) Völlig unproblematisch sind die einwertigen Elektrolyte Natrium, Kalium. Das zweiwertige Magnesium, welches sehr hydroskopisch ist, sehr gut dissoziiert und sehr leicht, so wie das Kalium, in die Weichlinse ein- und austreten kann; ist als Aktivitätsverbesserer erwünscht. Es ist ein guter Partner mit Carbonat und Hydrogencarbonat, aber auch mit Citrat und Chlorid. Beide sind sehr gute Negativ-Ionen-Partner. Am günstigsten ist es, wenn alle Elektrolyte in gleicher Menge vorhanden sind. Die Gesamtmenge der Salze sollte um 20% liegen. Eine völlige Sättigung bringt das Risiko des Auskristallisierens bei Temperaturschwankungen. Die Aktivität der dissoziierten Elektrolyte ist so am schwächsten. Liegt die schwammartige Hydroweichlinse in dieser Elektrolytlösung, so sollen die Elektrolyte möglichst oft in die Linse ein- und austreten, also das Wasser aus der Linse und in die Linse verschieben. So ist der Reinigungseffekt am besten. Ist die Lösung gesättigt, so ist ihre konservierende Wirkung am besten. Ein guter Kompromiss liegt bei einer Konzentration um 20%. Wird lediglich eine Konzentration um 10% verwendet, wird die Linse vom Linsenträger nicht als hart, sondern als weich empfunden, und es besteht die Gefahr, dass diese Linse aufgesetzt wird. Sie würde sich am Auge festsaugen und sehr beschwerlich sein.

G) Der pH-Wert um 9,0 und 4,0 kann nicht verwendet werden, da in diesem Bereich diese Salzlösung sehr aggressiv ist und das Linsenmaterial angreift. Ein leicht saures Milieu ist pilzfreundlich, ist also nicht geeignet, auch deswegen nicht, weil der pH der beschriebenen Abspüllösung tränenfilmidentisch sein sollte, im Idealfall bei 7,70 (leicht alkalisch), bei schwächstmöglicher Pufferkapazität (gibt an Hydrolinsen die beste Verträglichkeit). Es ist erwünscht, die Säure-Basen-Flut zwischen Aufbewahrungslösung und Abspüler (Auslauglösung) möglichst gross zu halten. Das gilt auch für das osmotische Druckgefälle. Auch Temperaturschwankungen sind günstig, um das hydrodynamische System möglichst optimal zu halten. Ein pH von etwa 5,0 ist ideal, ist auch pilzfeindlich. In der Hydrolinsenpflege ist das sehr wichtig. Dieser pH greift die Hydrolinsen nicht an.

Werden bestimmte pH-Werte für bestimmte Materialsorten gewünscht, so sind die Pufferbasen und Puffersäuren des Citrats sehr günstig. Tartrat- und Acetatpuffer sind ebenfalls sehr günstig. Im Prinzip sind alle biologischen Puffer geeignet. Der Phosphatpuffer kann unter Weglassen des Magnesiums verwendet werden.

Mögliche biologische Puffer sind:

| Puffersäure: | Pufferbase: |
| --- | --- |
| $H_2CO_3$ | $HCO_3^-$ |
| $CO_2$ | $HCO_3^-$ |
| $NH_4$ | $NH_3$ |
| $H_2PO_4^-$ | $HPO_4^-$ |
| Citrat $2^-$ | Citrat $3^-$ |
| Harnsäure | Urat |
| Milchsäure | Laktat |
| Essigsäure | Acetat |

Der beschriebene sterile, konservierende Hydroweichlinsenaufbewahrer, welcher frei von augenfremden Stoffen ist, der während seiner Funktion als Aufbewahrer auch ein hydrodynamischer Reiniger ist, hat vorzugsweise folgende Ausstattung:

I. Formel:

6,66% Natrium-Citrat
6,66% Kalium-Citrat
6,66% Magnesium-Carbonat (oder ersatzweise Hydrogencarbonat)
II. Formel:

**10% Natrium-Citrat / ersatzweise Hydrogencarbonat oder**

**10% Kalium-Chlorid / Carbonat**

III. Formel:
20% Natrium-Citrat
IV. Formel:
20% Natrium-Carbonat (oder ersatzweise Hydrogencarbonat)
V. Formel:
20% Natrium-Chlorid
VI. Formel:
20% Kalium-Citrat
VII. Formel:
20% Kalium-Carbonat (oder ersatzweise Hydrogencarbonat)
VIII. Formel:
20% Kalium-Chlorid.
IX. Formel:
20% Magnesium-Carbonat (oder ersatzweise Hydrogencarbonat)
X. Formel:
20% Magnesium-Citrat
XI. Formel:
20% Magnesium-Chlorid

Für den reinigenden, hydrodynamischen Effekt ist das Kräftegleichgewicht das günstigste. Es ist aber auch möglich, all diese Kombinationen so zu wählen, dass die Elektrolyte starke Kräfteunterschiede aufweisen, z.B.:
17% Natrium-Chlorid
2% Kalium-Citrat
1% Magnesium-Carbonat (Hydrogencarbonat)

In einem weniger zu bevorzugenden Beispiel können auch alle positiven Elektrolyte an denselben negativen Elektrolyt gebunden sein, z.B.:

**Natrium-Chlorid / ersatzweise alle an Citrat oder Carbonat**

**Kalium-Chlorid oder Hydrogencarbonat**

**Magnesium-Chlorid**

Alle weiteren möglichen Kombinationen mit diesen bevorzugten Elektrolyten sind möglich.
Die Zugabe von Calcium mit einem Chelatbildner wäre eine Möglichkeit, aber weniger gut.

Die Kombination mit einem geeigneten Abspüler:

Mit der Patentanmeldeschrift, Aktenzeichen P 3315974.2 wurde ein ungiftiger (nicht toxischer) Linsenabspüler bekannt, der frei von augenfremden Stoffen ist und deren Wahl an Elektrolyten präzise nach optimaler Nützlichkeit ausgesucht werden. Da viele Linsenträger an trockenen Augen leiden und Natrium-Chlorid der oder einer der Hauptverursacher von trockenen Augen ist, so wurde Natrium-Chlorid ausgegrenzt. Auch deshalb, weil hydratisierte Natrium-Ionen um 47% grösser sind als die Kalium-Ionen und 51% grösser als Chlorid-Ionen. Die hydratisiert kleineren Elektrolyte können in die Hydrolinse leichter ein- und austreten, regeln schneller den osmotischen Druck und die Isohydrie, durchspülen die Rückstände schneller. Auch das zweiwertige Magnesium entspricht diesem Konzept, zudem dissoziiert es besonders leicht und ist sehr hygroskopisch und ist als Beigabe zur Verbesserung der Aktivierung der hydrodynamischen Elektrolytlösung sehr wichtig. Physiologisch wirkt es beruhigend auf die Nerven, schützt vor Übersteuerung, ist ein guter Protein-Transporter (bindet dieses). Zur Pufferung dieser Lösung kommen alle biologisch bedeutsamen Puffersysteme in Frage, die in dieser Schrift aufgeführt sind; bevorzugt nach dem wichtigen Leitgedanken: Pufferkapazität so gering wie möglich. So ist die Lösung am verträglichsten. Zu bevorzugende Werte: 1/10 bis 1/15 sollte der Anteil der Puffersalze an den Isotonisierungssalzen sein. Das Problem der nicht dissoziierten Elektrolyte des Puffers ist so am geringsten. Diese treten in die Weichlinse nämlich weniger leicht ein und aus. Sind sie in grösseren Mengen vorhanden, so kann die Weichlinse nach dem Einsetzen zum Festsaugen neigen, besonders wenn diese physiologische Lösung mit der Augenbadewanne angewendet wird, in Verbindung Hydroweichlinse- Auge.

Die pH-Spezifikation dieser Lösung ist am günstigsten bei 7,70 und kann im physiologischen Bereich zwischen pH 7,0 und 7,8 liegen. Wenn der ideale pH des Aufbewahrers bei 5,0 liegt, bzw. im noch sehr günstigen Bereich 4,5-6,5, so besteht zwischen beiden Lösungen eine optimale Säure-Basen-Flut als hydrodynamische, wasserverschiebende Kraft. Dabei ist aus bereits erläuterten Gründen der biologische Phosphatpuffer nicht zu bevorzugen und es ist gut, auf ihn zu verzichten.

Die Abspüllösung - Auslauglösung kann so gestaltet werden, dass der Abspüler mit Kalium-Citrat (an Stelle von Chlorid) isotonisiert ist oder statt Citrat ganz oder teilweise Laktat. Dieser Abspüler kann wiederum auch einen Magnesiumanteil haben, der ganz oder teilweise an Chlorid, Citrat oder Laktat oder einem Gemisch dieser 3 Negativ-Elektrolyte gebunden ist. Der Effekt:Kochsalzgegenspieler ist auch so aufzubauen. Eine Calcium-Komponente mit Chelatbildner wäre möglich, ist aber nicht angezeigt.

Der so beschriebene Hydroweichlinsenabspüler ist steril verpackt und ist eine ideale Tränenfilmersatzlösung und kann auch als Augenbadewasser angewendet werden, auch zur Isotonisierung von Augentropfen, als lokale Elektrolyt-Therapie.

Ergeben sich während des Tragens durch Trockenheit am Auge Kontaktlinsentrageprobleme, so kann die sterile, isohydrische, isotonische Abspüllösung, die frei von körperfremden, augenfremden Stoffen mit einer Augenbadewanne oder durch Aufträufeln am Auge angewendet werden. Diese säubert die Linse und schafft Verträglichkeit und Sehschärfenverbesserungen, ohne dass die weiche oder hartflexible Linse abgesetzt werden muss. Toxische, künstliche Tränen passen nicht in dieses untoxische Pflege- und Trageprogramm, wegen ihrer unerwünschten toxischen Wirkungen.

In der nicht toxischen hypertonischen Aufbewahrungslösung, die frei von körperfremden, augenfremden Stoffen ist, kann sehr gut auch der Negativelektrolyt Acetat eingefügt werden. Das gilt auch für den genannten Abspüler. Beim Ansetzen der Elektrolytlösungen für die Reiniger-, Aufbewahrer- und Abspüllösungen sind die Elektrolyte so der Lösung zuzugeben, wie sie sich am besten lösen.

Beispiele:

Magnesium-Chlorid dissoziiert sehr gut
Magnesium-Citrat oder Carbonat/Hydrogencarbonat dissoziiert sehr schlecht
Kalium-Carbonat löst sich sehr leicht.
Ist also z.B. Magnesium, Kalium und die Gegenspieler Chlorid und Carbonat gewünscht, so erfolgt die Zugabe in das Wasser wie folgt:
Magnesium-Chlorid
Kalium-Carbonat, usw.

**Ansprüche**

1. Verfahren zur Pflege von weichen Kontaktlinsen, bei dem die Linse
- in einem hypertonisierten, mit Konservierungsmittel versehenen Tensid-Reiniger ohne Zusatz von Verdickungsmitteln und Phosphaten gereinigt,
- in einer wässrigen, von augenflüssigkeitsfremden Stoffen freien und mit 20 Gew.% im Tränenfilm enthaltener Salze hypertonisierten Lösung aufbewahrt und
- mit einer von augenflüssigkeitsfremden Stoffen freien, isotonischen Salzlösung nachgespült wird.
2. Verfahren nach Anspruch 1, dadurch gekennzeichnet, dass der Reiniger schwach hypertonisiert ist und als Konservierungsmittel Chlorhexidin und Thimerosal enthalten ist.
3. Verfahren nach Anspruch 1, dadurch gekennzeichnet, dass der Reiniger stark hypertonisiert ist und die Konservierungsmittel aus der hohen Salzkonzentration bestehen.
4. Verfahren nach Anspruch 3, dadurch gekennzeichnet, dass die Salzkonzentration 10 bis 18 Gew.% vorzugsweise 14 Gew.% beträgt.

5. Verfahren nach Anspruch 1, dadurch gekennzeichnet, dass die Linse mit der salzhaltigen Aufbewahrungslösung abgerieben und abgespült wird.

6. Verfahren nach Anspruch 1, dadurch gekennzeichnet, dass die isotonische Nachspüllösung nicht abgegossen, sondern nochmals zum Vorspülen nach der Oberflächenreinigung verwendet wird.

7. Verfahren nach Anspruch 1, dadurch gekennzeichnet, dass zur Hypertonisierung des Reinigers und der Aufbewahrungslösung die Elektrolyte Natrium, Kalium, Magnesium, Chlorid, Citrat, Carbonat, Hydrogencarbonat verwendet werden.

8. Verfahren nach Anspruch 1, dadurch gekennzeichnet, dass die isotonische Nachspüllösung mit Kalium-Citrat, ganz oder teilweise mit Lactat oder mit einer Kombination Chlorid-Citrat-Lactat isotonisiert ist.

9. Aufbewahrungslösung für Kontaktlinsen, gekennzeichnet durch ca. 20 Gew.% im Tränenfilm enthaltener Salze.

10. Reinigungslösung für Kontaktlinsen, gekennzeichnet durch 10 bis 18 Gew.% körpereigene Salze.

11. Mittelkombination zur Durchführung des Pflegeverfahrens nach einem der Ansprüche 1-8, gekennzeichnet durch einen hypertonisierten, mit Konservierungsmittel versehenen Tensid-Reiniger ohne Zusatz von Verdickungsmitteln und Phosphaten, eine wässrige, von augenflüssigkeitsfremden Stoffen freie und mit 20 Gew.% im Tränenfilm enthaltener Salz hypertonisierte Aufbewahrungslösung und mit einer von augenflüssigkeitsfremden Stoffen freie, isotonische Nachspüllösung.

12. Mittelkombination nach Anspruch 11, dadurch gekennzeichnet, dass die Konservierungsmittel im Reiniger aus einer hohen Salzkonzentration von 10 bis 18 Gew.% bestehen.

13. Verwendung der in Anspruch 5 definierten Nachspüllösung als Augenbadewasser oder Tränenersatzlösung.

14. Verwendung des in Anspruch 12 definierten Reinigers als Handwaschmittel vor der Manipulation von Kontaktlinsen.